# EUROPÄISCHE PATENTANMELDUNG

(11) **EP 4 361 264 A1**
(43) Veröffentlichungstag der Anmeldung: **01.05.2024**
(21) Anmeldenummer: 23206158.0
(22) Anmeldetag: 26.10.2023
(51) Int. Cl.: C12N 13/00, C12N 1/20, A23C 9/123, A23C 19/068, C12R 1/225, C12R 1/46

(54) **VERFAHREN ZUM EINSTELLEN DER EIGENSCHAFTEN VON STARTERKULTUREN**

(30) Priorität: 27.10.2022 DE 102022211432
(71) Anmelder: Deutsches Institut für Lebensmitteltechnik e.V., 49610 Quakenbrück (DE)
(72) Erfinder: Hertel, Dr., Christian, 49610 Quakenbrück (DE); Chanos, Dr., Panagiotis, 49610 Quakenbrück (DE); Stühmeier-Niehe, Corinna, 49610 Quakenbrück (DE)
(74) Vertreter: Taruttis, Stefan Georg

(57) **Zusammenfassung**

Verfahren zur Herstellung von Starterkulturen für die Herstellung von Milchprodukten mit vorbestimmten Eigenschaften, bei dem die Starterkulturen mit gepulsten elektrischen Feldern (PEF) behandelt sind, die aus rechteckigen, monopolaren Hochspannungspulsen einer Feldstärke von 0,2 bis 1 kV/cm mit einer Pulsdauer von 5 bis 8 µs bei einer Frequenz von 1 bis 21 Hz für 20 bis 80 Pulsen, für jeden Parameter jeweils ±15% oder ±10% oder ±5%, behandelt sind.

## Beschreibung

Die vorliegende Erfindung betrifft ein Verfahren zum Einstellen der Eigenschaften von Starterkulturen bei der Herstellung von Milchprodukten durch Fermentation von Milch. Starterkulturen, die Milchsäurebakterien umfassen oder daraus bestehen, werden mit dem Verfahren hinsichtlich ihrer Eigenschaften eingestellt, die sie bei der Herstellung von Milchprodukten auf deren Produkteigenschaften haben, insbesondere auf die Festigkeit und/oder Synärese, z.B. von Sauermilch-, Joghurt- und Kefirerzeugnissen, z.B. Joghurt, Kefir, und Käse, z.B. Frischkäse, Pasta Filata, Mozzarella. Entsprechend betrifft die Erfindung ein Verfahren zur Herstellung von Starterkulturen für die Herstellung von Milchprodukten, sowie ein Verfahren zur Herstellung von Milchprodukten mit den durch das Verfahren hergestellten bzw. eingestellten Starterkulturen und die Verwendung der mit dem Verfahren hergestellten Starterkulturen zur Herstellung von Milchprodukten durch Fermentation.

Für die Zwecke der Erfindung können Starterkulturen insbesondere Streptococcus, z.B. Streptococcus thermophilus, Lactobacillus, z.B. Lactobacillus delbruckii ssp. bulgaricus, Lactobacillus delbruekii ssp. lactis, Lactobacillus helveticus, Lactobacillus acidophilus, Lactobacillus casei, Lactobacillus kefir, Lactobacillus parakefir, Lactococcus, z.B. Lactococcus lactis ssp. lactis, Lactococcus lactis ssp. lactis biovar. diacetylactis, Lactococcus lactis ssp. cremoris, Leuconostoc, z.B. Leuconostoc mesenteroides ssp. cremoris, Bifidobacterium, z.B. Bifidobacterium lactis, und Mischungen von zumindest zweien dieser sein, bevorzugt eine Mischung aus Lactobacillus delbruckii ssp. bulgaricus und Streptococcus thermophilus. Das Verfahren hat den Vorteil, dass die Starterkulturen dazu eingestellt werden können, in der Fermentation der Herstellung von Milchprodukten vorbestimmte Produkteigenschaften der Milchprodukte zu steuern, insbesondere zu verstärken oder abzuschwächen.

Chanos et al., European Food Research and Technology (2020), 246: 621-630 beschreibt die Behandlung der Milchsäurebakterien Streptococcus thermophilus und Lactobacillus delbrückii ssp. bulgaricus mit gepulsten elektrischen Feldern (PEF), die aus einer Kombination einer elektrischen Feldstärke von 1 oder 3,67 kV/cm, einer Frequenz von 0,5 oder 4 Hz und einer Pulsanzahl von 5 oder 50 ausgewählt sind und aufgrund der verwendeten ELCRACK HPV-Anlage (Elea Vertriebs- und Vermarktungsgesellschaft mbH, Quakenbrück) einen exponentiellen Spannungsverlauf aufweisen. Die optimale Behandlung war PEF bei 4 Hz, 1kV/cm für 50 Pulse und führte zu einer Verkürzung der Lag-Zeit bei der Joghurtfermentation von 12 min. Für bestimmte PEF-Parameter konnte eine um 50% geringere Größe des Inokulums der Starterkultur verwendet werden und eine Beschleunigung der Ansäuerung beobachtet werden.

Das Patent DE 10 2012 201 822 B4 beschreibt zur Herstellung von Starterkulturen mit Milchsäurebakterien deren Behandlung mit gepulsten elektrischen Feldern einer Feldstärke von 7,5 bis 10 kV/cm, um eine stärkere Absäuerung bei der Reifung von Rohwurst zu erreichen.

Der Erfindung stellt sich die Aufgabe, ein alternatives Verfahren zur Behandlung von Starterkulturen, insbesondere zur Einstellung von deren Fermentationseigenschaften für Milchprodukte, bereitzustellen und ein Verfahren zur Herstellung von Milchprodukten anzugeben, bei dem die Produkteigenschaften durch Einstellung der Starterkulturen vorbestimmt werden. Bevorzugt sollen die Starterkulturen zu einem Milchprodukt mit geringer Synärese und hoher Festigkeit führen.

Die Erfindung löst die Aufgabe mit den Merkmalen der Ansprüche und stellt insbesondere ein Verfahren zur Herstellung von Starterkulturen bereit, die für die Herstellung von Milchprodukten mit vorbestimmten Eigenschaften dadurch eingestellt sind, dass die Starterkulturen mit gepulsten elektrischen Feldern (PEF) behandelt sind, die aus rechteckigen, monopolaren Hochspannungspulsen einer Feldstärke von 0,2 bis 1 kV/cm mit einer Pulsdauer von 5 bis 8 µs bei einer Frequenz von 1 bis 21 Hz bestehen, für 20 bis 80 Pulse, für jeden Parameter jeweils ±15% oder ±10% oder ±5%, z.B. einer Feldstärke von 0,6 kV/cm mit einer Pulsdauer von 7 µs bei einer Frequenz von 11 Hz für 50 Pulse, für jeden Parameter jeweils ±25% oder ±20% oder ±15% oder ±10%. Während der Behandlung mit PEF können die Starterkulturen in wässrigem Medium, z.B. Peptonwasser, Milch, Wasser oder isotonischer wässriger Lösung suspendiert sein. Optional werden die Starterkulturen nach der Behandlung mit PEF in demselben Medium bzw. ohne Änderung des Mediums, bevorzugt unmittelbar nach der Behandlung mit PEF, und insbesondere ohne Zusetzen eines Gefrierschutzmittels, eingefroren.

Die vorbestimmten Eigenschaften der mit den Starterkulturen hergestellten Milchprodukte sind ausgewählt unter Synärese, die bevorzugt geringer als die mit einer nicht mittels PEF behandelten Starterkultur gleicher Bakterien erzeugt ist, Festigkeit, die höher oder geringer als die mit einer nicht mittels PEF behandelten Starterkultur gleicher Bakterien erzeugt ist, und/oder der Geschmack. Bevorzugt ist das Milchprodukt Joghurt oder Käse, insbesondere Mozzarella. Entsprechend betrifft die Erfindung ein Verfahren zur Herstellung von Starterkulturen zur Verwendung in der Herstellung von Milchprodukten, sowie ein Verfahren zur Herstellung von Milchprodukten, wobei die Milchprodukte bevorzugt eine höhere Festigkeit und/oder eine geringere Synärese aufweisen, z.B. im Vergleich zu Milchprodukten, die unter gleichen Bedingungen hergestellt sind, jedoch mit denselben Bakterien als Starterkulturen, optional eingefroren, jedoch nicht mit PEF behandelt. Dabei ist das Milchprodukt bevorzugt Joghurt oder Käse, insbesondere Mozzarella. In der Ausführungsform, in der das Milchprodukt Mozzarella ist, kann das Milchprodukt optional eine geringere Festigkeit aufweisen und/oder einen weniger käsigen und/oder weniger sauren Geschmack.

Es hat sich gezeigt, dass durch die Behandlung von Starterkulturen mittels dieser gepulsten elektrischen Felder (PEF-Behandlung) bei der Fermentation von Milch Joghurt hergestellt wird, der im Vergleich mit einer Starterkultur derselben Bakterien jedoch ohne diese PEF-Behandlung eine höhere Festigkeit und/oder eine geringere Synärese aufweist. Die Lag-Phase bei der Ansäuerung und die erreichte Ansäuerung wurden durch die PEF-Behandlung nicht oder nur geringfügig beeinflusst und entsprachen der Lag-Phase und erreichten Ansäuerung, die bei der Fermentation mit nicht denselben Bakterien ohne vorherige PEF-Behandlung erreicht wurden.

Für die Herstellung von Joghurt sind für die PEF-Behandlung der Starterkultur gepulste elektrische Felder mit 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2), oder 1 kV/cm, 80 Pulsen bei 21 Hz, 8 µs Pulsdauer (PEF16), in jedem Fall aus rechteckigen, monopolaren Hochspannungspulsen bevorzugt. Bei der Milchfermentation zur Herstellung von Joghurt ergeben die mit gepulsten elektrischen Feldern PEF5 hergestellten Starterkulturen eine kürzere Lag-Phase, mit PEF6 Joghurt mit einer geringeren Synärese, mit PEF4 durch höhere proteolytische Aktivität Joghurt mit erhöhtem Gehalt an freien Aminosäuren, mit PEF2 Joghurt mit größerer Festigkeit. Diese Eigenschaften der Starterkulturen blieben auch nach Einfrieren in flüssigem Stickstoff und anschließender Lagerung bei -20 °C für 2 Wochen erhalten und erzeugten vergleichbare oder vorzugsweise dieselben Produkteigenschaften im Joghurt.

Für die Herstellung von Käse, z.B. Mozzarella, ist ein PEF-Behandlung der Starterkultur mit gepulsten elektrischen Feldern von 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2) bevorzugt. Im Vergleich mit einer nicht mit PEF behandelten Starterkultur ergibt eine mit PEF2 behandelte Starterkultur einen festeren Mozzarella mit milderem, weniger saurem und weniger käsigem Geschmack, eine mit PEF4 behandelte Starterkultur einen säurehaltigeren Geschmack, und eine mit PEF5 behandelte Starterkultur einen festeren Mozzarella mit im Wesentlichen unverändertem Geschmack, eine mit PEF6 behandelte Starterkultur einen weicheren Mozzarella mit im Wesentlichen unverändertem Geschmack.

Die mit dem Verfahren behandelten Starterkulturen haben den Vorteil, bei der Fermentation von Milch zur Herstellung von Joghurt eine höhere Festigkeit im Joghurt zu erzeugen, bevorzugt zusätzlich eine geringere Synärese.

Außerdem haben die mit dem Verfahren behandelten Starterkulturen den Vorteil, dass die mit ihnen durchgeführte Fermentation von Joghurt oder Käse, z.B. von Mozzarella, eine im Vergleich zu unbehandelten Starterkulturen kürzere Lag-Phase haben, z.B. kürzer um zumindest 20 min, bevorzugt um zumindest 25 min.

Generell hat sich gezeigt, dass die mit der PEF-Behandlung hergestellten Starterkulturen auch nach Einfrieren und/oder Gefriertrocknen die Fähigkeit im Wesentlichen beibehalten, die Produkteigenschaften von damit hergestellten Milchprodukten zur vorbestimmten Festigkeit und/oder geringerer Synärese zu erzeugen. Daher können die Starterkulturen nach der PEF-Behandlung eingefroren oder gefriertrocknet werden, gelagert werden, bevorzugt bei -20 °C oder darunter, z.B. in flüssigem Stickstoff, und anschließend zur Herstellung von Milchprodukten eingesetzt werden. Beim Einfrieren und Lagern kann der Starterkultur vor oder nach der PEF-Behandlung ein Gefrierschutzmittel zugesetzt werden, z.B. Glycerin.

Die Erfindung wird nun anhand von Beispielen mit Bezug auf die Figuren beschrieben, die in
- Fig. 1 Festigkeitswerte für Joghurt,
- Fig. 2 Werte zur Synärese von Joghurt,
- Fig. 3 Festigkeitswerte für Mozzarella
zeigen.

### Beispiel 1: Verfahren zur Herstellung von Starterkulturen für Joghurt

Als Starterkultur für Joghurt wurden Lactobacillus delbrueckii subsp. bulgaricus, Stamm LB186 bei 37 °C in mMRS-Bouillon, und Streptococcus thermophilus, Stamm ST504, bei 42 °C in M17-Bouillon separat unter Rühren (50 Upm) bis zum Erreichen einer OD₆₀₀ von 0,4 inkubiert, damit jeweils separate Medien angeimpft und für ca. 8 h inkubiert, bis Endkonzentrationen von ca. 3 × 10⁸ KBE/ml erreicht wurden. Die Kulturen wurden separat bei 10000 × g abzentrifugiert, zweimal in sterilem Peptonwasser gewaschen und anschließend in Peptonwasser suspendiert und zu gleichen Teilen gemischt, um eine Zellzahl von 2,5 × 10⁸ KBE/ml zu erhalten. Diese Suspension wurde zwischen parallelen, im Abstand von 20 cm angeordneten Elektroden (System PEF-Pilot Dual, Elea Vertriebs- und Vermarktungs-GmbH, Quakenbrück) ohne Bewegung der Suspension mit gepulsten elektrischen Feldern aus monopolaren, rechteckigen Hochspannungspulsen beaufschlagt, die in den folgenden PEF-Parametern variiert wurden:
Spannung: 4 kV (0,2 kV/cm), 20 kV (1 kV/cm) oder 12 kV (0,6 kV/cm),
Pulsanzahl: 20 oder 80 oder 50,
Frequenz: 1 Hz, 21 Hz oder 11 Hz,
Pulsdauer: 5 µs, 8 µs oder 7 µs.

Die so hergestellten Starterkulturen wurden entweder unmittelbar in Milch eingemischt oder ohne Zusatz, optional mit Zusatz eines Gefrierschutzmittels, in flüssigem Stickstoff eingefroren, bei -20 °C gelagert und aufgetaut ("gefroren") und anschließend in Milch eingemischt. Als Kontrollproben wurden Starterkulturen, die nicht mit PEF behandelt waren und optional gefroren waren, in Parallelversuchen mitgeführt.

Als Milch wurde 9 Gew.-% Magermilchpulver (SMP SH Basic Magermilchpulver, DMK GmbH, Zeven) in destilliertem Wasser gelöst, auf 90 °C erwärmt und vor dem Zusetzen der Starterkultur für 1 d bei 4 °C gelagert.

In einzelnen Bechern wurden 147 ml der Milch gefüllt, die auf 42 °C vorgewärmt war, und mit je einer Starterkultur zu einer Endkonzentration von 5 × 10⁶ KBE/ml versetzt. Die Becher wurden mit einem luftdichten Deckel verschlossen, durch den eine pH-Sonde in die Milch ragte. Die Becher, Parallelansätze für jede Analyse, wurden statisch bei 42°C inkubiert, wobei Temperaturunterschiede zwischen einzelnen Bechern vermieden wurden.

Der Verlauf der Ansäuerung, als Änderung des pH-Werts gemessen, zeigte keine signifikante Abhängigkeit von einem der PEF-Parameter.

Es wurde festgestellt, dass die PEF-Behandlung die Festigkeit des Joghurts signifikant beeinflusste:

| PEF-Parameter | Synärese | Festigkeit |
|---|---|---|
| **0,2 kV/cm, 80 Pulse bei 1 Hz, Pulsdauer 5 µs (PEF5)** | | |
| Kontrolle | 47.57 ± 0.40 | 0.65 ± 0.19 |
| PEF behandelt | 46.05 ± 0.91 | 1.05 ± 0.11 |
| Kontrolle + gefroren | 47.70 ± 0.41 | 0.57 ± 0.20 |
| PEF behandelt + gefroren | 45.15 ± 0.66 | 0.68 ± 0.11 |

| **0,2 kV/cm, 20 Pulse bei 1 Hz, 8 µs Pulsdauer (PEF2)** | | |
|---|---|---|
| Kontrolle | 46.21 ± 1.13 | 0.77 ± 0.22 |
| PEF behandelt | 42.35 ± 0.68 | 1.20 ± 0.16 |
| Kontrolle + gefroren | 44.52 ± 1.31 | 0.66 ± 0.03 |
| PEF behandelt + gefroren | 43.29 ± 0.71 | 0.82 ± 0.07 |

| **0,2 kV/cm, 20 Pulse bei 21 Hz, 8 µs Pulsdauer (PEF4)** | | |
|---|---|---|
| Kontrolle | 50.48 ± 2.77 | 0.82 ± 0.10 |
| PEF behandelt | 40.54 ± 0.95 | 1.25 ± 0.04 |
| Kontrolle + gefroren | 48.69 ± 1.09 | 0.66 ± 0.13 |
| PEF behandelt + gefroren | 41.55 ± 0.70 | 0.86 ± 0.10 |

| **0,2 kV/cm, 80 Pulse bei 1 Hz, Pulsdauer 8 µs (PEF6)** | | |
|---|---|---|
| Kontrolle | 46.58 ± 0.70 | 0.82 ± 0.8 |
| PEF behandelt | 41.10 ± 1.54 | 1.06 ± 0.073 |
| Kontrolle + gefroren | 46.79 ± 0.57 | 0.56 ± 0.06 |
| PEF behandelt + gefroren | 41.11 ± 0.47 | 0.73 ± 0.11 |

| **1 kV/cm, 80 Pulse bei 21 Hz, 8 µs Pulsdauer (PEF16)** | | |
|---|---|---|
| PEF behandelt | 36,78 ± 9,01 | 1,42 ± 0,47 |

Die Angaben sind Mittelwerte der Synärese und maximale Festigkeitswerte.

Die Festigkeit ist generell in N angegeben, gemessen als maximale Kraft (N) für das Eindringen einer zylindrischen Sonde, Durchmesser 36 mm, Eindringtiefe 10 mm, Vorschub mit 0,5 mm/s, Belastung 1 N (Ta.XT plus Texture Analyzer, Stable Microsystems, Goldaming, GB).

Die Synärese ist generell in % freigesetzter Flüssigkeit angegeben, gemessen durch Lagern der Probe bei -20 °C für maximal 7 Tage, Auftauen bei Raumtemperatur über 3 h, Abzentrifugieren bei 500 × g, 10 min bei 4 °C und Wiegen der freigesetzten Flüssigkeit. Die Synärese gibt das anteilige Gewicht freigesetzter Flüssigkeit zum verbleibenden Joghurt an.

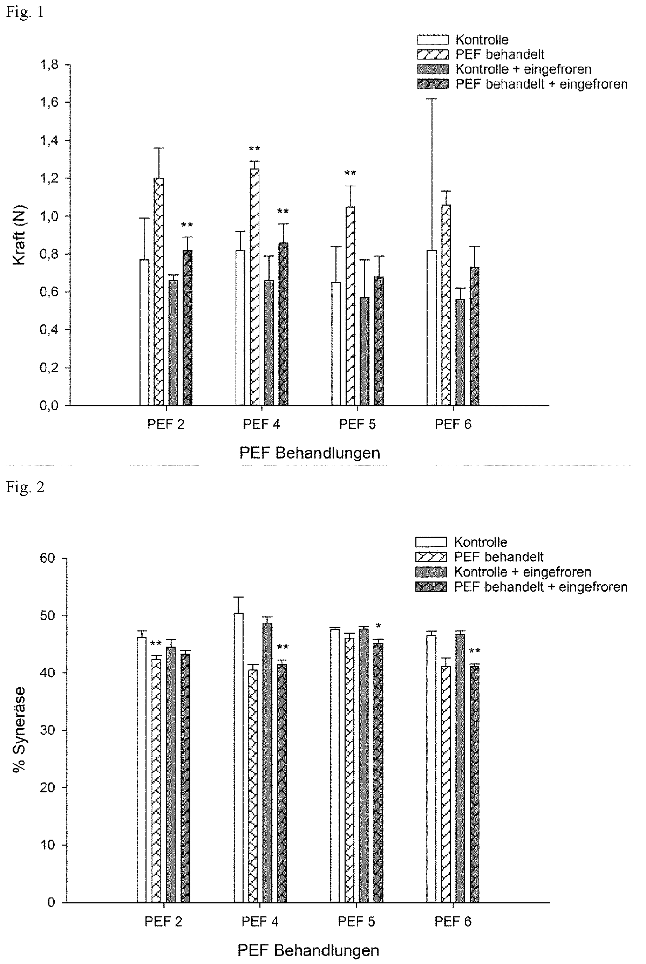

Die Fig. 1 zeigt Festigkeitswerte von Joghurt für verschiedene PEF-Behandlungen, die bei der Herstellung der Starterkulturen eingesetzt wurden. Dabei können nur jeweils die in einer Gruppe dargestellten Werte miteinander verglichen werden. Die Werte zeigen, dass die Behandlung der Starterkulturen mit PEF2, PEF4, PEF5 und PEF6 und dem anschließenden direkten Einmischen in Milch (PEF treated) zur Herstellung von Joghurt höherer Festigkeit als mit unbehandelter Starterkultur (Control) führte, und auch bei Einfrieren nach PEF-Behandlung (PEF treated + frozen) und anschließendem Einmischen in Milch Joghurt höherer Festigkeit hergestellt wurde, als mit Starterkulturen ohne PEF-Behandlung, die gleichermaßen eingefroren wurden (Control + frozen).

Die Fig. 2 zeigt den Einfluss der PEF-Behandlung auf die Synärese, bestimmt als Austritt von Flüssigkeit aus Joghurt. Die Werte zeigen, dass die Behandlung der Starterkulturen mit PEF2, PEF4, PEF5 und PEF6 nach direktem Einmischen in Milch (PEF treated) zur Herstellung von Joghurt geringerer Synärese als mit unbehandelter Starterkultur (Control) führte, und auch bei Einfrieren nach PEF-Behandlung (PEF treated + frozen) Joghurt geringerer Synärese hergestellt wurde, als mit Starterkulturen ohne PEF-Behandlung, die gleichermaßen eingefroren wurden (Control + frozen).

Die Ergebnisse zeigen, dass durch die PEF-Behandlung Starterkulturen hergestellt werden, die bei der Herstellung von Milchprodukten am Beispiel von Joghurt die Festigkeit (höher) und die Synärese (niedriger) günstig beeinflussen. Diese Eigenschaften der durch die PEF-Behandlung hergestellten Starterkulturen blieb auch nach Einfrieren und Lagerung bei -20°C für 14 d erhalten.

Weiterhin wurde festgestellt, dass die Behandlung mit PEF5 Starterkulturen erzeugt, die bei der Fermentation eine im Vergleich zu unbehandelten Starterkulturen um zumindest 20 min, bevorzugt eine um 21 min kürzere Lag-Phase haben.

### Beispiel 2: Verfahren zur Herstellung von Starterkulturen für Mozzarella

Die Starterkulturen wurden nach Beispiel 1 hergestellt.

Zylindrische Mozzarella-Proben wurden vor Beginn der Messung mindestens 30 min bei Raumtemperatur gelagert. Die zylindrische Sonde (36 mm Durchmesser) des Ta.XT plus Texture Analyzers bewegte sich mit einer Geschwindigkeit von 1 mm/s über eine Strecke von 8 mm.

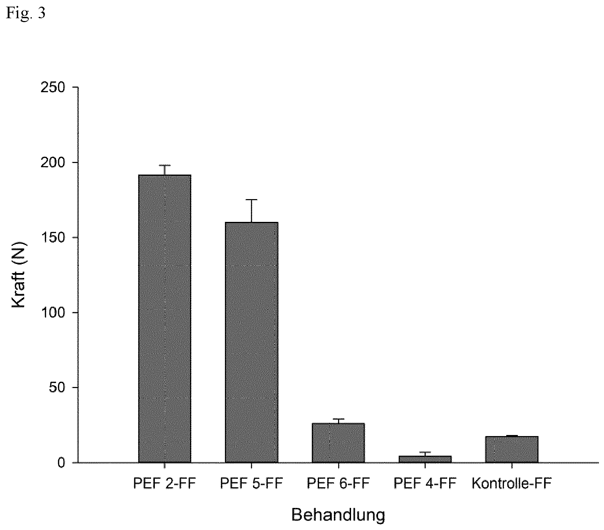

Die in Fig. 3 dargestellten Ergebnisse zeigten, dass sich die Festigkeit von Mozzarella-Käse, der mit PEF-behandelten Kulturen hergestellt wurde, signifikant von der Härte von Mozzarella-Käse unterscheidet, der mit der nicht behandelten Starterkultur (Control) hergestellt wurde. Während die mit den Kulturen PEF 2-FF, PEF 5-FF und PEF 6-FF zubereiteten Mozzarellas eine höhere Festigkeit hatten als die mit der Control-Kultur hergestellten, erzeugte die mit PEF4 behandelte Starterkultur weicheren Mozzarella als die Control-Kultur. Diese Ergebnisse stimmen mit der sensorischen Bewertung der Festigkeit überein, obwohl Mozzarella, der mit PEF6-behandelter Starterkultur auch bei der Messung signifikant fester war als der mit der Control-Kultur hergestellte.

Mit einer mit PEF2 behandelten Starterkultur hergestellter Mozzarella wurde im Vergleich zur Kontrolle als milder (weniger sauer/käsig) bewertet. Im Gegensatz dazu wurde einer mit PEF4 behandelten Starterkultur hergestellter Mozzarella als säurehaltiger bewertet als die Kontrolle. Bei den mit PEF 5 oder PEF 6 behandelten Starterkulturen hergestellten Mozzarellas wurden im Vergleich zur Control-FF keine signifikanten Unterschiede im Geschmack festgestellt. Was die wahrgenommene Festigkeit/Kaubarkeit betrifft, ergaben mit PEF 2-FF und PEF 5-FF behandelte Starterkulturen signifikant festere/kaubarere Mozzarellas als die Kontrolle. Im Gegensatz dazu ergab eine mit PEF 4 behandelte Starterkultur weicheren/weniger kaubaren Mozzarella als die Kontrolle. Die mit PEF 6 behandelte Starterkultur erzeugte keine signifikanten Unterschiede in Bezug auf Festigkeit und Kaueigenschaften zur Kontrolle.

Dieses Ergebnis zeigt, dass die Herstellung von Starterkulturen mit Behandlung durch gepulste elektrische Felder Eigenschaften der Starterkulturen erzeugt, die bei der Herstellung von Käse, hier stellvertreten durch Mozzarella, gezielt zu anderen Festigkeiten und wahlweise weniger käsigem Geschmack führt.

## Patentansprüche

1. Verfahren zur Herstellung von Starterkulturen zur Verwendung bei der Herstellung von Milchprodukten durch Behandlung von Starterkulturen mit gepulsten elektrischen Feldern (PEF), die aus rechteckigen, monopolaren Hochspannungspulsen einer Feldstärke von 0,2 bis 1 kV/cm mit einer Pulsdauer von 5 bis 8 µs bei einer Frequenz von 1 bis 21 Hz bestehen, für jeden Parameter jeweils ±15%, für 20 bis 80 Pulse.

2. Verfahren nach Anspruch 1, **dadurch gekennzeichnet, dass** die Starterkulturen nach der Behandlung mit gepulsten elektrischen Feldern eingefroren oder gefriergetrocknet werden.

3. Verfahren nach einem der Ansprüche 1 bis 2, **dadurch gekennzeichnet, dass** die Starterkultur ausgewählt ist unter Streptococcus thermophilus, Lactobacillus, z.B. Lactobacillus delbruckii ssp. bulgaricus, Lactococcus, z.B. Lactococcus lactis ssp. lactis, Leuconostoc, z.B. Leuconostoc cremoris, Bifidobacterium, z.B. Bifidobacterium lactis, und Mischungen von zumindest zweien dieser.

4. Verfahren nach einem der voranstehenden Ansprüche, **dadurch gekennzeichnet, dass** die Behandlung mit PEF bei 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2), oder 1 kV/cm, 80 Pulsen bei 21 Hz, 8 µs Pulsdauer (PEF16), für jeden Parameter jeweils ±15%, erfolgt.

5. Verfahren zur Herstellung eines Milchprodukts durch Fermentation von Milch mit einer Starterkultur, **dadurch gekennzeichnet, dass** die Starterkultur nach einem Verfahren gemäß einem der voranstehenden Ansprüche hergestellt ist.

6. Verfahren nach Anspruch 5, **dadurch gekennzeichnet, dass** das Milchprodukt Joghurt ist, wobei Joghurt durch die Behandlung der Starterkulturen mit PEF eine höhere Festigkeit und eine geringere Synärese aufweist als unter ansonsten gleichen Bedingungen mit nicht PEF-behandelten Starterkulturen hergestellter Joghurt.

7. Verfahren nach Anspruch 5, **dadurch gekennzeichnet, dass** das Milchprodukt Mozzarella ist, wobei Mozzarella durch die Behandlung der Starterkulturen mit PEF eine höhere Festigkeit aufweist als unter ansonsten gleichen Bedingungen mit nicht PEF-behandelten Starterkulturen hergestellter Mozzarella.

8. Verfahren nach einem der Ansprüche 5 bis 7, **dadurch gekennzeichnet, dass** die Milch durch Lösen von Milchpulver in Wasser erzeugt ist.

9. Verfahren nach einem der Ansprüche 5 bis 8, **dadurch gekennzeichnet, dass** die Behandlung mit PEF bei 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2), oder 1 kV/cm, 80 Pulsen bei 21 Hz, 8 µs Pulsdauer (PEF16), für jeden Parameter jeweils ±15%, erfolgt und dass das Milchprodukt Joghurt ist.

10. Verfahren nach einem der Ansprüche 5 bis 8, **dadurch gekennzeichnet, dass** die Behandlung mit PEF bei 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2), oder 1 kV/cm, 80 Pulsen bei 21 Hz, 8 µs Pulsdauer (PEF16), für jeden Parameter jeweils ±15%, erfolgt und dass das Milchprodukt Mozzarella ist.

11. Verfahren nach einem der Ansprüche 5 bis 8, **dadurch gekennzeichnet, dass** die Behandlung mit PEF bei 0,2 kV/cm, 20 Pulsen bei 21 Hz, 8 µs Pulsdauer (PEF4) oder 0,2 kV/cm, 80 Pulsen bei 1 Hz, Pulsdauer 5 µs (PEF5), oder 0,2 kV/cm, 80 Pulsen bei 1 Hz, Pulsdauer 8 µs (PEF6), oder 0,2 kV/cm, 20 Pulsen bei 1 Hz, 8 µs Pulsdauer (PEF2), oder 1 kV/cm, 80 Pulsen bei 21 Hz, 8 µs Pulsdauer (PEF16), für jeden Parameter jeweils ±15%, erfolgt.
